# EUROPEAN PATENT APPLICATION

(11) **EP 4 775 990 A1**
(43) Date of publication of application: **15.07.2026**
(21) Application number: 25305033.0
(22) Date of filing: 13.01.2025
(51) Int. Cl.: G01N 33/574

(54) **NOVEL T CELL POPULATIONS AS MARKERS FOR PREDICTING TOXICITY AND EFFECTIVENESS OF IMMUNE CHECKPOINT BLOCKADE-BASED THERAPIES**

(71) Applicant: Commissariat à l'Energie Atomique et aux Energies Alternatives, 75015 Paris (FR); Assistance Publique - Hôpitaux de Paris, 75012 Paris (FR); Université Paris Cité, 75006 Paris (FR)
(72) Inventor: LEMAOULT, Joël, 75010 Paris (FR); ROUAS-FREISS, Nathalie, 75010 Paris (FR); DUMONT, Clément, 75010 Paris (FR); CULINE, Stéphane, 75010 Paris (FR)
(74) Representative: Gevers & Orès

(57) **Abstract**

The present invention relates to the field of anticancer treatment and concerns new populations of T cells, as blood markers to predict toxicity or effectiveness of immune checkpoint blockade (ICB)-based therapy in patients with cancer.

## Description

The present invention relates to the field of anticancer treatment and concerns new populations of T cells, as blood markers to predict toxicity or effectiveness of immune checkpoint blockade (ICB)-based therapy in patients with cancer.

ICB is a novel immunotherapeutic strategy that boosts anti-cancer immune responses by targeting immunologic receptors on the surface of T cells. This approach has been found to improve outcomes in the treatment of a variety of cancers with long-lasting antitumor results. In particular, immunotherapy based on ICB has now become essential in the medical management of advanced urological cancers, particularly clear cell renal cell carcinoma (ccRCC) and urothelial carcinoma (UC). It is now being offered as a first-line treatment, most often as part of combinations with other anticancer drugs, to more and more patients. The ICB currently available in France in these indications mainly involves nivolumab and pembrolizumab targeting the lymphocyte inhibitory receptor PD-1 (Programmed cell Death protein 1), as well as ipilimumab targeting another inhibitory receptor, CTLA-4 (Cytotoxic T-Lymphocyte Associated protein 4).

In advanced ccRCC, patients with adverse prognostic factors may be treated with either a "dual" ICB associating an anti-PD-1 (nivolumab) and an anti-CTLA-4 (ipilimumab), with a 42% objective response rate and a durable long-term response, or a combination of an anti-PD-1 (nivolumab or pembrolizumab) with an oral antiangiogenic drug (axitinib, cabozantinib, lenvatinib), with higher immediate objective response rates but possibly less durable response.

In advanced UC, the new standard-of-care first-line treatment is a combination of pembrolizumab with enfortumab vedotin, an antibody-drug-conjugate designed for the treatment of cancer expressing Nectin-4, with an overall response rate of 67%. Although the latter is a potent anticancer drug, its side effects (e.g. peripheral neuropathy, diabetes) preclude long-term use and may be irreversible, and the long-term benefit of this combination will probably remain associated with pembrolizumab activity.

However, despite the indisputable clinical benefits of ICB-based therapy, its use is associated with a wide spectrum of side effects that require early detection and proper management. Indeed, ICB often cause specific toxicities, called immune-related adverse events (or irAEs), which may involve any organ or system of the body. Frequently, gastrointestinal, dermatologic, hepatic, endocrine, and pulmonary toxicities predominate. In general, ICB therapy can continue in the presence of mild irAEs with close monitoring, but moderate to severe irAEs may be associated with life-threatening declines in organ function and quality of life, and fatal outcomes have been reported. Consequently, it is important that patients with a low likelihood of response are spared from the potential toxicity of ICB, if they could be reliably identified before treatment initiation.

Currently, there are no validated markers predictive of the occurrence of toxicity and only insufficient biomarkers predictive of the effectiveness of ICB in patients with advanced urological cancer, which would constitute valuable tools to help oncologists in therapeutic choices. Instead, current practice is limited to routine monitoring and management of significant and possibly life-threatening ICB toxicities. Although much explored, assessment of PD-L1 protein expression does not reliably predict response to treatment at the individual patient level. The search for new biomarkers is therefore a clinical necessity. Hence, predicting immunotoxicity risks alongside response could provide a personalized risk-benefit profile, inform therapeutic decision-making, and improve clinical trial cohort selection.

In this context, there thus remains a genuine need for means to anticipate, in a patient suffering from cancer, the toxicity and efficacy of ICB immunotherapy in order to guide the therapeutic choice based on these parameters.

The present invention is believed to meet such a need by providing a new non-invasive blood marker which can be easily used in clinical routine and easily measurable in laboratory routine.

Surprisingly, the Inventors have discovered a new subpopulation of T cells identified by the combination of the following surface markers: CD3, CD8 and NKp80, preferably in combination with CD57 and CD45RA, and whose blood levels are predictive of the risk of ICB toxicity (CD3⁺CD8⁺CD57⁺CD45RA⁺NKp80⁺) and effectiveness (CD3⁺CD8⁺CD57⁺CD45RA⁺NKp80⁻) in patients with advanced urological cancer.

This novel blood marker is particularly advantageous in that it constitutes a theragnostic tool to guide the therapeutic choice in order to avoid administration of ICB-based therapy to a poor responder patient or to a patient at high risk of irAEs.

In a main aspect, the present invention thus relates to a method of monitoring a subject, comprising determining *in vitro* the presence or level of a T cell population in a biological sample from the subject, said T cell population being either CD3⁺ CD8⁺ NKp80⁺ or CD3⁺ CD8⁺ NKp80⁻.

The method of the invention can be used for monitoring a human subject treated, or likely to be treated, with an ICB-based therapy.

As used herein, the expression "immune checkpoint blockade-based therapy" (or ICB-based therapy) designates a treatment with any drug, molecule or composition which blocks an immune checkpoint. In particular, it includes, but is not limited to, anti-PD1 antibodies, anti-PD-L1 antibodies, anti-CTLA-4 antibodies and anti-LAG3 antibodies. Although the currently used drugs blocking immune checkpoints are monoclonal antibodies, other molecules specifically binding to PD1, PD-L1, CTLA-4 or LAG3 could be used for the development of future ICB-based therapies such as, for example, antibody fragments or specifically designed aptamers. Of course, the phrases "anti-PD1", "anti-PD-L1", "anti-CTLA-4" and "anti-LAG3" encompass any therapy with active molecules that antagonize PD1 or PD-L1, CTLA-4 or LAG3.

A further aspect of the invention relates to a method for predicting the toxicity of an ICB-based therapy in a subject, comprising determining, in a biological sample from the subject, the level of a T cell population that is CD3⁺ CD8⁺ NKp80⁺, said level being predictive of the toxicity of the ICB-based therapy.

As used herein, the term "toxicity" designates the occurrence or the risk of side effects or immune-related adverse events in a subject administered with a therapy. In particular, it designates the occurrence or the risk of an "Immune-related adverse event".

As used herein, the expression "Immune-related adverse event" (or irAE) designates a range of side-effects and complications commonly associated with the use of immune-checkpoint inhibitors. The clinical spectrum of irAEs is vast and almost every organ system may be affected. Frequent irAEs include, but are not imited to, gastrointestinal, endocrine, and dermatologic toxicities. Fatal irAEs include, but are not limited to, neurotoxicity, cardiotoxicity, and pulmonary toxicity. The occurrence of irAEs is usually higher in immune-checkpoint inhibitors (ICI) combination therapy than in ICI monotherapy.

Preferably, the irAE is selected from the group comprising thyroiditis, arthritis, lipase increased or pancreatitis, colitis, pneumopathy or pneumonitis, hypophysitis, dry mouth & dry eye, pericarditis, myocarditis, myositis, type 1 diabetes, nephritis and bullous dermatitis.

The method of the invention can be used to predict the health hazard for a subject before administering an ICB-based therapy to said subject as first line therapy, or to predict the health hazard for a subject who already received an ICB-based therapy as first line therapy.

A further aspect of the invention relates to a method for predicting the effectiveness of an ICB-based therapy in a subject, comprising determining, in a biological sample from the subject, the level of a T cell population that is CD3⁺ CD8⁺ NKp80⁻, said level being predictive of the effectiveness of the ICB-based therapy.

As used herein, the term "effectiveness" means that a subject administered with a therapy shows a positive response to the therapy provided. Non-limiting examples of such a positive response can be clinical benefits, defined by either objective response (tumor shrinkage) or stable disease, progression-free survival or overall survival.

The method of the invention can be used to predict the responsiveness of a subject before administering an ICB-based therapy to said subject as first line therapy, or to predict the responsiveness of a subject who already received an ICB-based therapy as first line therapy.

The following embodiments apply *mutatis mutandis* to any of the methods of the invention. In other words, what is explained for a method of the invention is applicable to a method of monitoring a subject and a method for predicting the toxicity or the effectiveness of an ICB-based therapy.

In an embodiment, the invention relates to a method as defined above, further comprising determining whether said T cell population is CD57⁺.

In an embodiment, the invention relates to a method for predicting the toxicity of an ICB-based therapy in a subject as defined above, comprising determining, in a biological sample from the subject, the level of a T cell population that is CD3⁺ CD8⁺ NKp80⁺ CD57⁺, said level being predictive of the toxicity of the ICB-based therapy.

In an embodiment, the invention relates to a method for predicting the effectiveness of an ICB-based therapy in a subject as defined above, comprising determining, in a biological sample from the subject, the level of a T cell population that is CD3⁺ CD8⁺ NKp80⁻ CD57⁺, said level being predictive of the effectiveness of the ICB-based therapy.

In an embodiment, the invention relates to a method as defined above, further comprising determining whether said T cell population is CD45RA⁺.

In an embodiment, the invention relates to a method for predicting the toxicity of an ICB-based therapy in a subject as defined above, comprising determining, in a biological sample from the subject, the level of a T cell population that is CD3⁺ CD8⁺ NKp80⁺CD45RA⁺, said level being predictive of the toxicity of the ICB-based therapy.

In an embodiment, the invention relates to a method for predicting the effectiveness of an ICB-based therapy in a subject as defined above, comprising determining, in a biological sample from the subject, the level of a T cell population that is CD3⁺ CD8⁺ NKp80⁻ CD45RA⁺, said level being predictive of the effectiveness of the ICB-based therapy.

In an embodiment, the invention relates to a method for predicting the toxicity of an ICB-based therapy in a subject as defined above, comprising determining, in a biological sample from the subject, the level of a T cell population that is CD3⁺ CD8⁺ NKp80⁺ CD57⁺ CD45RA⁺, said level being predictive of the toxicity of the ICB-based therapy.

In an embodiment, the invention relates to a method for predicting the effectiveness of an ICB-based therapy in a subject as defined above, comprising determining, in a biological sample from the subject, the level of a T cell population that is CD3⁺ CD8⁺ NKp80⁻ CD57⁺ CD45RA⁺, said level being predictive of the effectiveness of the ICB-based therapy.

In an embodiment, the invention relates to a method as defined above, wherein the ICB-based therapy blocks the interactions of at least one target protein selected from the group comprising PD-1, PD-L1, LAG-3 and CTLA-4 and combinations thereof.

In an embodiment, the invention relates to a method as defined above, wherein the ICB-based therapy consists of at least one drug that antagonizes at least one target protein selected from the group comprising PD-1, PD-L1, LAG-3 and CTLA-4 and combinations thereof.

In an embodiment, the invention relates to a method as defined above, wherein the ICB-based therapy consists of at least one antibody selected from the group comprising anti-PD1 antibody, anti-PD-L1 antibody, anti-LAG-3 antibody and anti-CTLA-4 antibody and combinations thereof.

In an embodiment, the invention relates to a method as defined above, wherein the anti-PD1 antibody is selected from the group comprising nivolumab, pembrolizumab, cemiplimab, pidilizumab, lambrolizumab, balstilimab, sasanlimab, MEDI-0680, PDR001, REGN2810, combinations, generics and biosimilars thereof.

In an embodiment, the invention relates to a method as defined above, wherein the anti-PD-L1 antibody is selected from the group comprising atezolizumab, durvalumab, avelumab, BMS-936559, YW243.55.570, combinations, generics and biosimilars thereof.

In an embodiment, the invention relates to a method as defined above, wherein the anti-CTLA4 antibody is selected from the group comprising ipilimumab, tremelimumab, combinations, generics and biosimilars thereof.

In an embodiment, the invention relates to a method as defined above, wherein said at least one antibody is selected from the group comprising nivolumab, pembrolizumab and ipilimumab and combinations thereof.

In an embodiment, the invention relates to a method as defined above, wherein the ICB-based therapy consists of at least one multispecific antibody.

A multispecific antibody can recognize two or more epitopes located on the same or distinct targets.

In an embodiment, the invention relates to a method as defined above, wherein the ICB-based therapy consists of at least one multispecific antibody which blocks the interactions of at least one, preferably at least two, target proteins selected from the group comprising PD-1, PD-L1, LAG-3 and CTLA-4 and combinations thereof.

In an embodiment, the invention relates to a method as defined above, wherein the ICB-based therapy consists of at least one multispecific antibody which blocks the interactions of several target proteins selected from the group comprising PD-1, PD-L1, LAG-3 and CTLA-4 and combinations thereof.

In an embodiment, the invention relates to a method as defined above, wherein the ICB-based therapy consists of at least one multispecific antibody which blocks the interactions of at least PD-1 and/or PD-L1 and at least one other target protein.

In an embodiment, the invention relates to a method as defined above, wherein the ICB-based therapy is a combination of at least two antibodies, preferably a combination of nivolumab and ipilimumab.

In an embodiment, the invention relates to a method as defined above, wherein the subject has a cancer.

As used herein, "cancer" means all types of cancers. Indeed, the immune system plays a dual role against cancer since it prevents tumor cell outgrowth and also sculpts the immunogenicity of the tumor cells. Drugs blocking an immune checkpoint can hence be used to treat virtually any type of cancer. Also, the toxicity of IBD-based treatments is similar regardless the type of cancer.

In particular, a cancer according to the invention can be solid or non-solid cancers. Non limitative examples of cancers are carcinomas or adenocarcinomas such as urological cancer (including urothelial carcinoma), breast cancer, prostate cancer, ovary cancer, lung cancer, kidney cancer, pancreas cancer or colon cancer, sarcomas, lymphomas, melanomas, leukemias, germ cell cancers and blastomas.

In an embodiment, the invention relates to a method as defined above, wherein the cancer is selected from the group comprising urological cancer, breast cancer, prostate cancer, ovary cancer, lung cancer, kidney cancer, pancreas cancer or colon cancer, sarcomas, lymphomas, melanomas, leukemias, germ cell cancers and blastomas, preferably urological cancers.

In a preferred embodiment, the invention relates to a method as defined above, wherein the cancer is an urological cancer selected from clear cell renal cell carcinoma (ccRCC) and urothelial carcinoma (UC).

In a preferred embodiment, the cancer is an advanced cancer.

As used herein, the expression "advancer cancer" designates a cancer that has spread from the original (primary) site or has come back (recurred).

As used herein, the expression " biological sample" designates any sample of biological fluid or tissue from a subject, preferably a sample of blood, plasma, serum, urine, or saliva. Before subjecting the sample to the test according to the invention, the sample may be pretreated in accordance with standard processing techniques known in the art for each type of the collected sample (such as blood, urine or saliva). The sample may also be aliquoted and stored in a glass or plastic container, e.g., a tube or syringe, etc., without or with appropriate preservative. The sample may be stored in a 4°C refrigerator for short-term storage. The sample may also be frozen in a -80°C freezer for long-term storage, as long as necessary for carrying out the method according to the invention.

In an embodiment, the invention relates to a method as defined above, wherein the biological sample is a sample of biological fluid or tissue, preferably a sample of blood, plasma, serum, urine or saliva, more preferably a sample of blood.

The presence or level or amount of T cell populations in a sample may be determined by using standard techniques known *per se* in the art, such as flow cytometry or various immunodiagnostic assays such as immunoassay (e.g., enzyme immunoassay ELISA, a sandwich immunoassay, radioassay, radioimmunoassay (RIA), enzymatic assay, $, etc).

The detection methods generally include revealing labels such as fluorescent, chemiluminescent, radioactive, enzymatic labels or dye molecules, or other reactions for detecting the formation of a complex between the antigen and the antibody or antibodies reacted therewith.

In an embodiment, the invention relates to a method as defined above, wherein the presence or level of the T cell population is determined by flow cytometry, radioassay, enzymatic assay, immunoassay or radioimmunoassay.

In a preferred embodiment, determination of the level of the T cell population is carried out by flow cytometry. The basic principle of flow cytometry is the passage of cells in single file in front of a laser so they can be detected, counted and sorted. Cell components are fluorescently labelled and then excited by the laser to emit light at varying wavelengths. The fluorescence can then be measured to determine the amount and type of specific T cells present in a sample. For example, determination of the level of T cell populations expressing the phenotype CD3⁺CD8⁺NKp80⁺CD57⁺CD45RA⁺ or CD3⁺CD8⁺NKp80⁻CD57⁺CD45RA⁺ may be carried out by flow cytometry using any monoclonal antibody anti-CD3, anti-CD8, anti-NKp80, anti-CD57 and anti-CD45RA known in the art.

In an embodiment, the invention relates to a method as defined above, wherein a high level of T cell population with the phenotype CD3⁺CD8⁺NKp80⁺, preferably CD3⁺CD8⁺NKp80⁺CD57⁺, more preferably CD3⁺CD8⁺NKp80⁺CD45RA⁺, indicates that the ICB-based therapy for the subject is at high risk of toxicity for the subject.

In an embodiment, the invention relates to a method as defined above, wherein a high level of T cell population with the phenotype CD3⁺CD8⁺NKp80⁻, preferably CD3⁺CD8⁺NKp80⁻CD57⁺, more preferably CD3⁺CD8⁺NKp80⁻CD45RA⁺, indicates that the subject is more likely to respond to the ICB-based therapy.

As used herein, the expression "high level" means that the level of T cell population of interest is superior to a predetermined threshold. Of course, the skilled artisan can identify or refine thresholds, depending on the technique used to measure the relative abundance of the T cell population. Also, the threshold to be considered when performing the above methods can be predetermined by measuring the abundance of the T cell population of interest in a representative cohort of individuals treated by an immune checkpoint blockade therapy, and whose response to this treatment and immune toxicities are known.

In another aspect, the present invention also relates to a kit comprising a CD3 binding reagent, a CD8 binding reagent and a NKp80 binding reagent, preferably packaged in different containers.

In an embodiment, the invention relates to a kit as defined above, further comprising at least one reagent selected from the group comprising a CD57 binding reagent and/or a CD45RA binding reagent.

In an embodiment, the invention relates to a kit as defined above, wherein the binding reagents are antibodies.

In an embodiment, the invention relates to a kit as defined above, further comprising reagents for an immunological detection test and, possibly, an instruction manual.

In another aspect, the present invention also relates to a composition comprising T cell and an excipient or diluent or culture medium, said T cell cells being either CD3⁺ CD8⁺ NKp80⁺ or CD3⁺ CD8⁺ NKp80⁻.

In an embodiment, the invention relates to a composition as defined above, wherein said T cells further are CD57⁺.

In an embodiment, the invention relates to a composition as defined above, wherein said T cells further are CD45RA⁺.

In another aspect, the invention also relates to a composition as defined above for testing *in vitro* the toxicity or the efficacity of an ICB-based therapy.

The following Examples are put forth so as to provide those of ordinary skill in the art with a complete disclosure and description of how to make and use the present invention, and are not intended to limit the scope of what the Inventors regard as their invention nor are they intended to represent that the experiments below are all or the only experiments performed. While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, material, composition of matter, process, process step or steps, to the objective, spirit and scope of the present invention. All such modifications are intended to be within the scope of the claims appended hereto.

### FIGURE LEGENDS

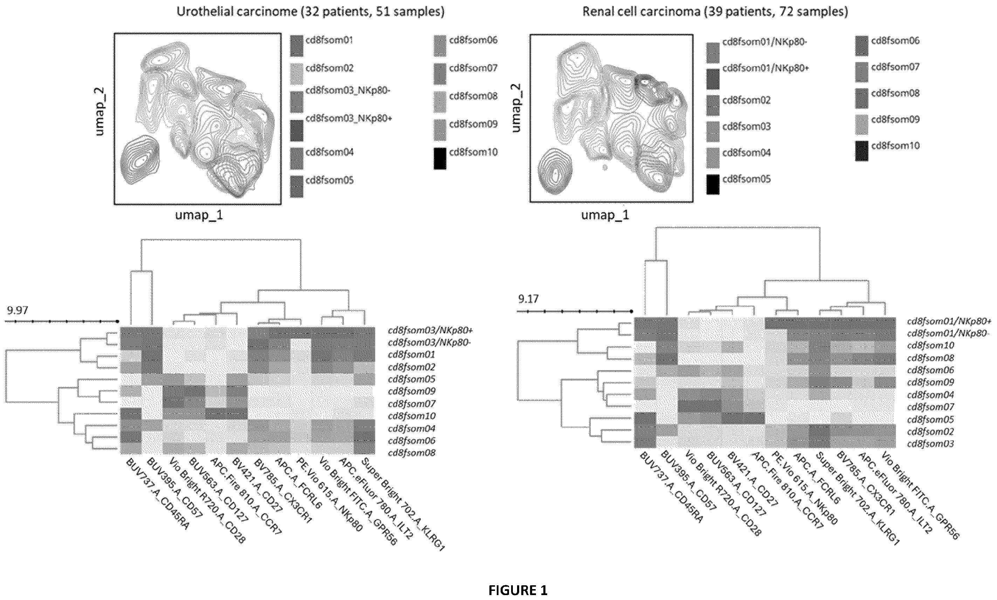

**Figure 1****.** Definition of CD8⁺ T cell clusters in patients treated with ICB for advanced urological malignancies.

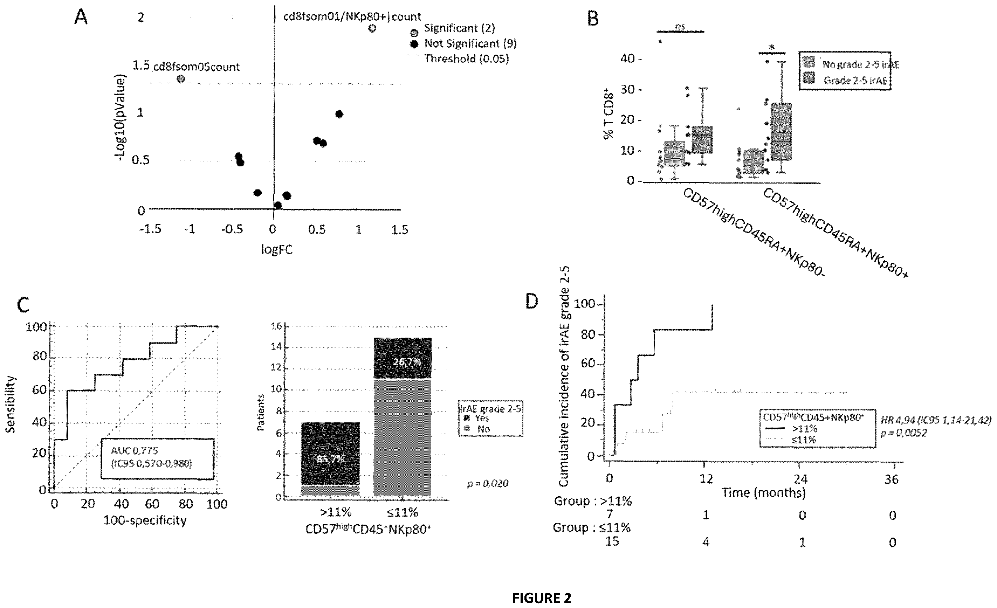

**Figure 2****.** Relevance of CD57⁺CD45RA⁺NKp80⁺CD8⁺ T cells in predicting immune-related adverse events in patients treated with combination nivolumab + ipilimumab. (A) EdgeR analysis of CD8+ T cell clusters predictive of grade 2-5 irAE. It revealed an association between pretreatment abundance of subcluster 01/NKp80+ among total CD8+ T cells and occurrence of grade 2-5 irAE. (B) Pretreatment quantification of CD57⁺CD45RA⁺NKp80⁺ CD8⁺ T in patients with and without grade 2-5 irAEs on nivolumab + ipilimumab. The proportion of CD57⁺CD45RA⁺NKp80⁺ cells among total CD3⁺CD8⁺CD4⁻ T cells in pretreatment blood samples was significantly higher in patients with ulterior occurrence of grade 2-5 irAE. (C) Determination of threshold values for CD57⁺CD45RA⁺NKp80⁺ CD8⁺ T cells associated with occurrence of grade 2-5 irAEs. ROC curve analysis was performed to establish relevant threshold values for the proportion of CD57+CD45RA+NKp80+ cells among total CD3+CD8+CD4- T cells in predicting ulterior occurrence of grade 2-5 irAE as a binary endpoint . Occurrence of grade 2-5 irAE was significantly more frequent in patients with above-threshold proportions of CD57⁺CD45RA⁺NKp80⁺ cells in both cohorts. (D) Time-dependent incidence of grade 2-5 irAE in patients on nivolumab + ipilimumab with above- and below-threshold CD57⁺CD45RA⁺NKp80⁺ CD8⁺ T cell counts. Timely-incidence of grade 2-5 irAEs was significantly higher in patients with above-threshold proportions of CD57⁺CD45RA⁺NKp80⁺ cells.

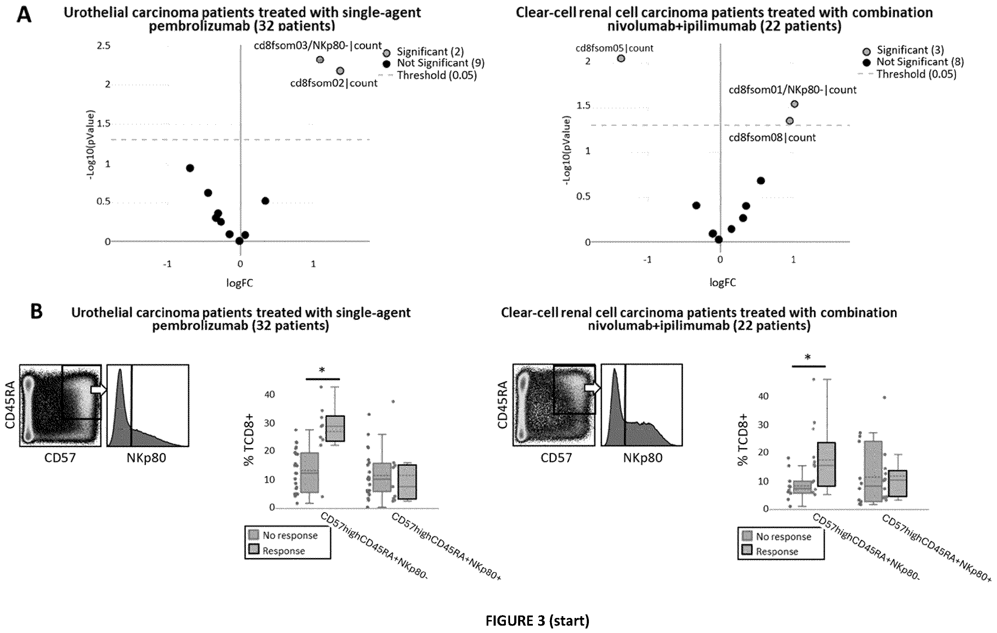

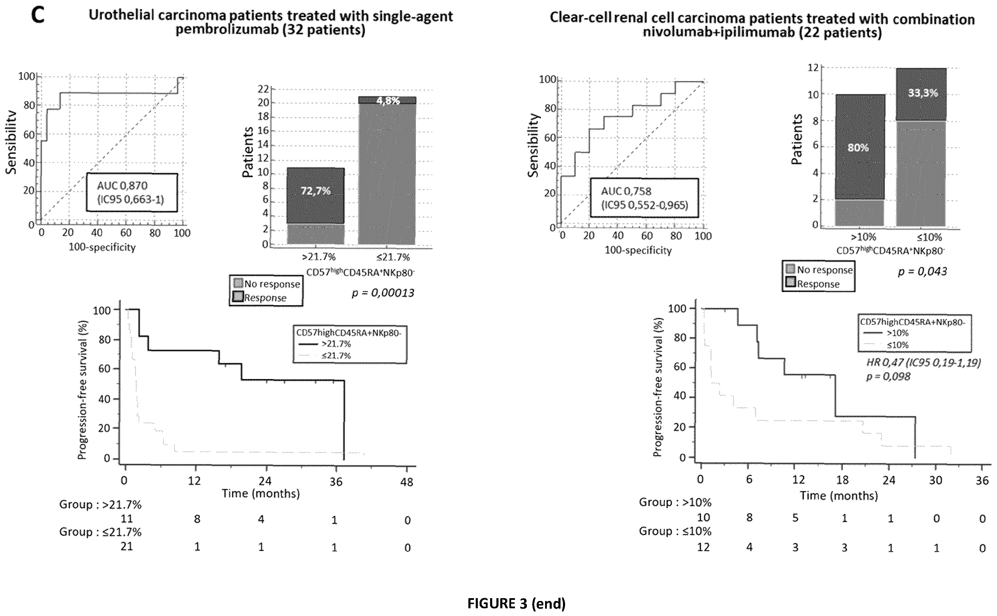

**Figure 3****.** Prognostic significance of CD57⁺CD45RA⁺NKp80⁻CD8⁺ T cells in patients treated with ICB for advanced urological malignancies. (A) EdgeR analysis of pretreatment CD8⁺ T cell clusters predictive of clinical benefit from immunotherapy. It revealed an association between pretreatment abundance of CD57⁺CD45RA⁺NKp80⁻ clusters and clinical response to immune checkpoint blockade in two independent groups of advance urological cancer patients. (B) Gating and pretreatment quantification of CD57⁺CD45RA⁺NKp80⁻ T cells in patients with and without clinical tumor response to ICB. Manual gating of CD57⁺CD45RA⁺NKp80⁻ cells is shown. The proportion of CD57⁺CD45RA⁺NKp80⁻ cells among total CD3⁺CD8⁺CD4⁻ T cells in pretreatment blood samples was significantly higher in patients with ulterior clinical response to immune checkpoint blockade. (C) Determination of threshold values for CD57⁺CD45RA⁺NKp80⁻CD8⁺ T cells associated with improved clinical outcomes. ROC curve analysis using clinical response as a binary endpoint was performed in both cohorts to establish relevant threshold values for the proportion of CD57⁺CD45RA⁺NKp80⁻ cells among total CD3⁺CD8⁺CD4⁻ T cells in predicting clinical outcomes. Clinical response was significantly more frequent in patients with above-threshold proportions of CD57⁺CD45RA⁺NKp80⁻ cells in both cohorts (bar plots). Progression-free survival was significantly better in pembrolizumab-treated urothelial carcinoma patients with above-threshold proportions of CD57⁺CD45RA⁺NKp80⁻ cells ; a non-significant similar trend was observed in renal-cell carcinoma patients.

### EXAMPLES

### Patients & Methods

### Patients & treatments

Adult patients with advanced solid cancer were enrolled in the single-center observational study GEIA (NCT04300088). The study was approved by an independent ethics committee. All patients provided written consent for biological sampling and clinical data collection. Patients were treated with either single-agent anti-PD-1 immunotherapy or the combination of nivolumab (anti-PD1) plus ipilimumab (anti-CTLA4) according to current standard-of-care. Assessment of response, stability or progression was performed by the investigators as per routine practice clinical and radiological information (investigator assessed-response). Progression-free survival was defined as the time between immunotherapy initiation and the occurrence of investigator-assessed progression or death, whichever occurred first. Overall survival was defined as the time between immunotherapy initiation and death from any cause. Cancer-specific survival was defined as the time between immunotherapy initiation and death from cancer progression; patients who died from other causes (including adverse events from treatments) were censored at the date of their death. Assessment of adverse events was performed using the Common Terminology Criteria for Adverse Events (CTCAE) v5.0. Immune-related adverse events (irAEs) were defined as AEs similar to autoimmune responses in the opinion of the investigator.

### Samples

Peripheral blood sampling was performed before the first infusion of immunotherapy and then on the day of subsequent infusions immediately preceding radiological assessments of tumor response. Healthy blood donors served as controls. Peripheral blood mononuclear cells (PBMC) were isolated using Ficoll gradient (Ficoll-Paque, LifeSciences) as per the manufacturer's instructions and stored at -150°C.

### Cell staining and spectral cytometry acquisition

After unfreezing, PBMCs were resuspended in 100µL of phosphate-buffered saline (PBS) completed with adequate buffer (Super Bright Complete Staining Buffer, eBioscience), human polyclonal immunoglobulin and a viability dye. After 5 minutes, multistep cell-surface staining was performed using a custom panel of 30 fluorochromes. This panel focused on effector T cells and comprised cell-surface markers for lymphocyte lineage, differentiation, cytotoxicity, activation and regulation (immune checkpoints). The complete list of targets and reagents used for staining is available in **Table 1.**

**Table 1. Cell-surface targets and reagents used for spectral cytometry.**

| **Marker** | **Fluorochrome** | **Clone** | **Manufacturer** |
|---|---|---|---|
| CD57 | Brilliant Ultraviolet 395 | NK-1 | BD Biosciences |
| Live Dead | Fix Blue | NA | Thermo Fisher Scientific |
| CD4 | Brilliant Ultraviolet 496 | SK3 | Thermo Fisher Scientific |
| CD127 | Brilliant Ultraviolet 563 | eBioRDR5 | Thermo Fisher Scientific |
| CD69 | Brilliant Ultraviolet 615 | FN50 | Thermo Fisher Scientific |
| CD45RA | Brilliant Ultraviolet 737 | HI100 | Thermo Fisher Scientific |
| CXCR4 | Brilliant Ultraviolet 805 | 12G5 | BD Biosciences |
| CD27 | Brilliant Violet 421 | O323 | BioLegend |
| CXCR3 | Pacific Blue | G025H7 | BioLegend |
| TCRgd | Brilliant Violet 480 | B1 | BD Biosciences |
| CD19 | Brilliant Violet 510 | HIB19 | BioLegend |
| HLA-DR | Brilliant Violet 570 | L243 | BioLegend |
| TCR Va7.2 | Brilliant Violet 605 | 3C10 | BioLegend |
| TIM-3 | Brilliant Violet 650 | F38-2E2 | BioLegend |
| KLRG1 | SuperBright 702 | 13F12F2 | Thermo Fisher Scientific |
| CX3CR1 | Brilliant Violet 785 | 2A9-1 | BioLegend |
| GPR56 | VioBright FITC | REA467 | Miltenyi Biotec |
| CD8 | Alexa Fluor 532 | RPA-T8 | Thermo Fisher Scientific |
| CD3 | Spark Blue 574 | UCHT1 | BioLegend |
| CD38 | PerCP | HIT2 | BioLegend |
| CD16 | PerCP-Cy5.5 | 3G8 | BioLegend |
| human IgG4 | PE | HP6023 | SouthernBiotech |
| NKp80 | PE-Vio615 | REA845 | Miltenyi Biotec |
| PD-1 | PE-Fire 700 | A17188B | BioLegend |
| LAG-3 | PE-Cy7 | 3DS223H | Thermo Fisher Scientific |
| NKp30 | PE-Fire 810 | P30-15 | BioLegend |
| FCRL6 | APC | 7B7/FcRL6 | BioLegend |
| CD56 | Alexa Fluor 660 | TULY56 | Thermo Fisher Scientific |
| CD28 | VioBright R720 | REA612 | Miltenyi Biotec |
| ILT2 | APC-eFluor780 | HP-F1 | Thermo Fisher Scientific |
| CCR7 | APC-Fire 810 | G043H7 | BioLegend |

Data acquisition was performed on a 5-laser Cytek Aurora spectral cytometer. Daily calibration and quality control was required before acquisition to ensure reproducibility of acquisition between batches. Reproducibility of staining was assessed visually during acquisition of each individual sample using the Spectroflo software (Cytek). Spectral data unmixing was performed with Spectroflo using single-stained cell controls.

### Data analysis

Data analysis was performed using Omiq (app.omiq.ai). Gating on lymphocytes, single cells and live cells was done manually for each individual sample. Additional compensation after spectral unmixing was performed for each acquisition batch; homogeneity of staining levels between batches was assessed visually. Selection of relevant T cell populations was done using manual gating.

After gating on live CD19-CD3+CD8+CD4- T cells, and exclusion of γδT cells (TCRγδ+) and MAIT (TCR Vα7.2+), UMAP dimension reduction and FlowSom clustering were performed using selected differentiation markers (CD3, CD8, CCR7, CD45RA, CD127, CD27, CD28, CD57, KLRG1, ILT2, GPR56, FcRL6, NKp80); clusters including NKp80+ cells were divided manually into NKp80- and NKp80+ subclusters.

Exploratory analysis of associations between cell cluster abundances (percentage of total CD8⁺ T cells) and clinical outcomes was performed using the EdgeR method (Robinson et al., Bioinformatics 26(1):139-140, 2010).

Spectral flow cytometry and multiparameter clustering are tools ill-suited to large-scale clinical application and validation as a routine biomarker. Thus, key markers that may be used to identify these relevant clusters in routine conventional flow cytometry assays were identified.

Analysis of clinical outcomes was performed using MedCalc. Associations with binary outcomes were analyzed using Fisher's exact text. Survival data was displayed with Kaplan-Meier curves and analyzed using the Logrank test.

### Example 1 - Analysis of patients and data

### Patients

Seventy three patients with urological cancer were included in the GEIA study. Forty-one patients with clear-cell renal cell carcinoma (ccRCC) and 32 patients with urothelial carcinoma (UC) were included. Patient characteristics at immunotherapy initiation are displayed in **Table 2.** Briefly, patients within the GEIA study had demographical and disease characteristics aligned with the epidemiology of their respective cancer. Treatments were administered as per current guidelines. Overall, response rates and toxicity were in line with pivotal trial data.

**Table 2. Characteristics of patients. FGFR: fibroblast growth factor receptor; IMDC: international metastatic renal cell carcinoma database consortium; NSCLC: non-small-cell lung cancer; UC: urothelial carcinoma; VEGFR-TKI: tyrosine kinase inhibitor targeting the vascular endothelial growth factor receptor; Bajorin risk factor score: performance status (0-1 vs 2+) and presence of visceral metastases (none vs one vs two or patients with liver metastases).**

| Tumor histology | | Renal cell carcinoma | Urothelial carcinoma |
|---|---|---|---|
| Number | | 41 | 32 |
| Age: median (extremes) | | 67 (42-85) | 67 (47-97) |
| Sex: | | | |
| Female | | 5 | 8 |
| Male | | 36 | 24 |
| Primary site (for UC): | | | |
| Bladder (±urethra) | | - | 20 |
| Upper tract (±bladder) | | - | 12 |
| Previous local treatment of primary tumor: | | | |
| Surgery | | 19 | 18 |
| Radiotherapy | | 0 | 4 |
| None | | 22 | 10 |
| Previous systemic treatments: | | | |
| Chemotherapy | | 0 | 32 |
| FGFR inhibitor | | 0 | 1 |
| VEGFR-TKIs | | 16 | 0 |
| None | | 25 | 0 |
| Metastatic sites: | | | |
| Locally advanced only | | 0 | 2 |
| Lymph node-only | | 2 | 7 |
| Visceral | | 39 | 23 |
| *Incl. liver metastases* | | *9* | *5* |
| *Incl. bone metastases* | | *10* | *10* |
| *Incl. brain metastases* | | *2* | *1* |
| Performance status: | | | |
| 0 | | 15 | 7 |
| 1 | | 14 | 12 |
| 2+ | | 12 | 12 |
| NA | | 0 | 1 |
| Prognostic classifiers at immunotherapy initiation | | IMDC risk: | Bajorin risk factor score: |
| | | Low: 2 | 0: 6 |
| | | Intermediate: 26 | 1: 14 |
| | | Poor: 13 | 2: 12 |
| Immunotherapy & response | | | |
| Nivolumab+ipilimumab | | 23 | 0 |
| | ➢ Response | Response: 13 (57%) | - |
| Single-agent anti-PD1 | | 18 | 32 |
| | ➢ Response | Response: 5 (28%) | 9 (28%) |

### Immunotherapy & outcomes

Twenty-three patients with ccRCC received the combination nivolumab+ipilimumab; all other patients received single-agent anti-PD1. Investigator-assessed response was observed in 18 patients with ccRCC (44%), including 13/23 patients on nivolumab+ipilimumab (57%) and 5/18 patients on single-agent anti-PD1 (28%), as well as in 9/32 patients with UC on single-agent anti-PD1 (28%) (**Table 2**). At last follow-up, 16 of 23 ccRCC patients treated with nivolumab+ipilimumab had experienced disease progression, 11 of whom received at least one subsequent line of treatment. Ten of these 16 patients had died from disease progression. One other patient had died shortly after immunotherapy initiation without confirmation of disease progression; one patient had died of surgical complications after cytoreductive nephrectomy in the context of complete response of metastatic disease after immunotherapy.

Twenty-four of 32 UC patients treated with single-agent anti-PD-1 had experienced disease progression and 18 had died.

Grade 2 to 4 (i.e. clinically relevant) immune-related adverse events (irAEs) following ICB treatment are shown in **Table 3.** There were no grade 5 (lethal) AEs related to immunotherapy in the whole study.

**Table 3. Grade 2-4 immune-related adverse events. irAE: immune-related adverse event.**

| | Single-agent anti-PD-1 (n=50) | Nivolumab+ipilimumab (n=23) |
|---|---|---|
| Patients with at least 1 grade 2-4 irAE | 10 (20%) | 11 (48%) |
| Thyroiditis | 2 | 0 |
| Arthritis | 2 | 0 |
| Lipase increased or pancreatitis | 2 | 0 |
| Colitis | 1 | 3 |
| Pneumopathy or pneumonitis | 1 | 1 |
| Hypophysitis | 1 | 1 |
| Dry mouth & dry eye | 1 | 0 |
| Pericarditis | 1 | 0 |
| Myocarditis | 0 | 2 |
| Myositis | 0 | 1 |
| Type 1 diabetes | 0 | 1 |
| Nephritis | 0 | 1 |
| Bullous dermatitis | 0 | 1 |

### Spectral flow cytometry phenotyping describes distinct clusters of CD8⁺ T cells

Samples from 39 ccRCC patients (72 samples) and 32 urothelial carcinoma patients (51 samples) were used for CD8⁺ T cell clustering by spectral flow cytometry in two separate analyses (**Figure 1**). T cell clustering showed good reproducibility between analyses.

### Example 2. Baseline CD8⁺ effector T cell clusters are predictive of toxicity from dual checkpoint blockade

Twenty-two ccRCC patients treated with nivolumab+ipilimumab with analyzable data from baseline PBMCs were included in a discovery analysis of clusters associated with toxicity from dual checkpoint blockade, focusing on CD8⁺ T cells prior to treatment (baseline). EdgeR analysis revealed that abundance of subcluster 01/NKp80⁺ relative to the whole of CD8⁺ T cells was associated with occurrence of grade 2-4 irAE analyzed as a binary parameter.

Heatmap analysis of differentiation markers showed that this was defined by strong expression of all markers for late-stage differentiation, effector functions and cytotoxicity, as well as by expression of the mature NK-cell Killer-Activating Receptor NKp80 (**Figure 1**). Thus, in order to provide a biomarker applicable to conventional cytometry-based assays using fewer markers, conventional gating of CD8⁺ T cells was performed to identify the CD57⁺CD45RA⁺NKp80⁺ subset of CD8⁺ T cells using the same dataset. ROC curve analysis determined a 11% threshold associated with 60% sensitivity and 91.7% specificity for incidence of grade 2-4 irAE as a binary event (85.7% vs 26.7%, p=0.020)(Figure 2C). Time-dependent incidence of grade 2-4 irAE was significantly higher in patients with >11% CD57⁺CD45RA⁺NKp80⁺ cells (**Figure 2D**).

In summary, we here describe an association between the abundance of pre-treatment late-differentiated cytotoxic CD8⁺ effector T cells and toxicity from anti-PD-1 + anti-CTLA-4 combination immunotherapy.

### Example 3. Baseline CD8⁺ effector T cell clusters are predictive of efficacy from both dual checkpoint blockade and single-agent anti-PD-1

A discovery analysis of pre-treatment CD8+ T cell clusters associated with clinical benefit from immune checkpoint blockade was performed independently in 32 patients treatment with single-agent pembrolizumab (anti-PD-1) for advanced urothelial carcinoma and 22 patient treated with nivolumab + ipilimumab (anti-PD-1 + anti-CTLA-4) for advanced renal-cell carcinoma.

EdgeR analysis revealed two clusters in each cohort that were associated with clinical response (**Figure 3A**). These clusters were phenotypically close within and between the two cohorts, with a phenotype that could be summarized as CD57⁺CD45RA⁺NKp80⁻ (**Figure 1**).Thus, in order to provide a biomarker applicable to conventional cytometry-based assays using fewer markers, conventional gating was performed to identify the CD57⁺CD45RA⁺NKp80⁻ subset of CD8⁺ T cells using the same dataset, as described in previous paragraphs (**Figure 3B**).

In urothelial carcinoma patients, ROC curve analysis revealed a 21.7% threshold associated with 88.9 sensitivity and 97% specificity for clinical response (response rates: 72.7% vs 4.8%, p=0.00013) (**Figure 3C**). Additionally, progression-free survival was significantly better in patients with higher baseline abundance of CD57⁺CD45RA⁺NKp80⁻ CD8⁺ T cells.

In renal-cell carcinoma patients, ROC curve analysis determined a 10% threshold with a 66.67% sensitivity and 80% specificity for clinical response (response rates: 80% vs 33.3%, p=0.043) (**Figure 3C**). Progression free survival was non-significantly better in patients with higher baseline abundance of CD57⁺CD45RA⁺NKp80⁻CD8⁺ T cells.

There were not enough patients with response in the smaller cohort of ccRCC patients treated with single-agent anti-PD-1 to allow for statistical analysis.

In conclusion, enrichment of CD8⁺ T cells from the peripheral blood in CD45RA+CD57⁺NKp80⁻ « early » cytotoxic effectors before ICB initiation, which can easily assessed with routine conventional flow cytometry equipment and analysis, is associated with response to immune checkpoint blockade and survival in two independent subsets of the GEIA cohort:
- ccRCC treated with anti-PD-1 + anti-CTLA-4 combination immunotherapy,
- Urothelial carcinoma treated with single-agent anti-PD-1.

## Claims

1. A method of monitoring a subject, comprising determining *in vitro* the presence or level of a T cell population in a biological sample from the subject, said T cell population being either CD3⁺ CD8⁺ NKp80⁺ or CD3⁺ CD8⁺ NKp80⁻.

2. A method for predicting the toxicity of an immune checkpoint blockade (ICB)-based therapy in a subject, comprising determining, in a biological sample from the subject, the level of a T cell population that is CD3⁺ CD8⁺ NKp80⁺, said level being predictive of the toxicity of the ICB-based therapy.

3. A method for predicting the effectiveness of an ICB-based therapy in a subject, comprising determining, in a biological sample from the subject, the level of a T cell population that is CD3⁺ CD8⁺ NKp80⁻, said level being predictive of the effectiveness of the ICB-based therapy.

4. The method according to any one of claims 1 to 3, further comprising determining whether said T cell population is CD57⁺.

5. The method according to any one of claims 1 to 4, further comprising determining whether said T cell population is CD45RA⁺.

6. The method according to any one of claims 2 to 6, wherein the ICB-based therapy blocks the interactions of at least one target protein selected from the group comprising PD-1, PD-L1, LAG-3 and CTLA-4 and combinations thereof.

7. The method according to any one of claims 2 to 6, wherein the ICB-based therapy consists of at least one antibody selected from the group comprising anti-PD1 antibody, anti-PD-L1 antibody, anti-LAG-3 and anti-CTLA-A antibody, generics, biosimilars and combinations thereof.

8. The method according to claim 7, wherein said at least one antibody is selected from the group comprising nivolumab, pembrolizumab and ipilimumab and combinations thereof.

9. The method according to any one of claims 2 to 8, wherein the ICB-based therapy is a combination of at least two antibodies, preferably a combination of nivolumab and ipilimumab.

10. The method according to any one of claims 1 to 9, wherein the subject has a cancer.

11. The method according to claim 10, wherein the cancer is selected from the group comprising wherein the cancer is selected from the group comprising urological cancer, breast cancer, prostate cancer, ovary cancer, lung cancer, kidney cancer, pancreas cancer or colon cancer, sarcomas, lymphomas, melanomas, leukemias, germ cell cancers and blastomas, preferably urological cancers.

12. The method according to claim 10 or 11, wherein the cancer is an urological cancer selected from clear cell renal cell carcinoma (ccRCC) and urothelial carcinoma (UC).

13. A kit comprising a CD3 binding reagent, a CD8 binding reagent and a NKp80 binding reagent, preferably packaged in different containers.

14. The kit comprising according to claim 13, further comprising at least one reagent selected from the group comprising a CD57 binding reagent and/or a CD45RA binding reagent.

15. The kit comprising according to claim 13 or 14, wherein the binding reagents are antibodies.
